# EUROPEAN PATENT APPLICATION

(11) **EP 0 648 478 A2**
(43) Date of publication of application: **19.04.1995**
(21) Application number: 94202901.8
(22) Date of filing: 07.10.1994
(51) Int. Cl.: A61F 2/34

(54) **Non-spherical acetabular cup for total hip replacement**

(30) Priority: 18.10.1993 US 137641
(71) Applicant: BRISTOL-MYERS SQUIBB COMPANY, New York, N.Y. 10154-0037 (US)
(72) Inventor: Farling, Gene M., Warsaw, IN 46580 (US); Shetty, Ravindranath H., Warsaw, IN 46580 (US); Ottersberg, Walter H., Warsaw, IN 46580 (US); Hori, Roy Y., Warsaw, IN 46580 (US)
(74) Representative: Mays, Julie

(57) **Abstract**

The device is a prosthetic cup prosthesis providing a surface for articulation of the spherical head of a joint arthroplasty. The articulating surface of the cup is the surface of revolution of an arc intersecting the cup equatorial plane and its polar axis, having a center of curvature translated from the spherical center of the cup, rotated about the cup's polar axis.

## Description

### Field of the Invention

This invention relates to prosthetic acetabular cups as used in total hip replacement surgery and has special application to a prosthetic acetabular cup having a non-spherical interior cavity.

### Background of the Invention

Up to the 1950s, hip prostheses in the United States consisted mainly of femoral endoprostheses, and a few cups designed to fit over the decapitated femoral neck as an articulating surface replacement for the necrotic femoral head. In most cases, the acetabulum was left untouched in hip replacement. The need to replace affected acetabulums forced the evolution of endoprostheses and femoral head replacements, and led to the development of metal acetabulum prostheses into which metal femoral endoprostheses would be articulated. Designs of the McKee-Farrar-type, Ring, and Sbarbaro total hip prostheses to address eroded and necrotic acetabulums as well as femoral heads are examples of the spectrum of devices that became available. These devices were cemented into their respective sites forming a total hip arthroplasty.

While the McKee-Farrar, et al., filled the perceived needs at that point in time, certain shortcomings of the all-metal arthroplasties were becoming apparent. Squeaking noises emanating from the hip in poorly lubricated arthroplasties, clicking noises due to the neck of the endoprosthesis striking the lip of the metal cup, and a sensation on the part of the patient of dragging due to the relatively high coefficient of friction between the prosthetic femoral head and acetabular cup were the most prevalent comments about these types of prostheses.

In an effort to overcome the shortcomings of the all-metal prosthesis, a British surgeon, Sir John Charnley, developed the archetype of low-friction arthroplasties, articulating a stainless steel femoral prosthesis in a polymeric acetabular prosthesis. The prosthesis's low-friction qualities were easily shown in vitro by moving the polymer cup over the metal prosthetic head. Movement of the head in the polymer cup was smoother and required perceptibly less force to initiate motion than the all-metal prosthesis.

The reliability of non-self lubricating articulating surfaces depends strongly on their ability to form reliable lubricating films on their surfaces. In a theoretical analysis of lubrication performance in non-polymeric total hip joints ("Analysis of Lubrication Performance in Metal-to-Metal Total Hip Joint [sic]," by Herbert S. Cheng and Alan Ai), the authors concluded that cup geometry, surface finish and, to a certain extent, the flexural modulus of the cup, affected the quality of a lubricating film protecting the articulating surfaces. The authors used a spherical model, in which the internal diameter of the cup and external diameter of the articulated femoral head were close to equal, and a model with the internal geometry of the Zimmer McKee-Farrar, in which the articulated femoral head rides on a thin, congruent band around the interior wall of the cup. One of their conclusions was that the spherical model would provide the environment for the most effective lubricating film if the surfaces were finished to a smoothness beyond the current, practical state-of-the-art in metal finishing. As to the McKee-Farrar model, the authors concluded that the presence of intersecting surfaces in the McKee-Farrar cup allows the super finishing of a very narrow circumferential band at the point of tangency of the head and cup. This was achieved in production by lapping the heads and cups together and offering them as a set that could not be interchanged. Another feature of the McKee-Farrar that makes it slightly superior to the congruent model in the generation of a stable lubricating film is the presence of entraining and escaping angles on either side of the head-cup tangent line. Since the direction of rotation of the head in the cup changes twice in the normal walking cycle, the roles of the angles are reversed on opposite sides of the cup. Generation of a reliably maintained lubricating film is marginally possible since the entraining angle on one side of the tangent line is not equal to the escaping angle into the superior space above the femoral head. The entraining angle is required to be shallow and equal to the escaping angle. In that sense, the obtuse and acute angles on opposite sides of the tangent line and the reversal of the roles of the angles on the opposite side of the head only marginally establish a reliable lubricating film.

### Summary of Invention

The device is a prosthetic cup prosthesis providing a surface for articulation of the spherical head of a joint arthroplasty. The articulating surface of the cup is the surface of revolution of an arc intersecting the cup equatorial plane and its polar axis, having a center of curvature translated from the spherical center of the cup, rotated about the cup's polar axis.

Accordingly, it is an advantage of the invention to provide a novel prosthetic acetabular cup for use in total hip replacement.

Another object of the invention is to provide for a prosthetic acetabular cup having a non-spherical inner cavity.

Another object of the invention is to provide for a metal acetabular cup for mating with a metal hip stem prosthesis.

Still other objects of the invention will become apparent upon a reading of the following description taken along with the accompanying drawings.

### Brief Description of the Drawings

Fig. 1 is a schematic representation of the acetabular cup geometry of the subject invention.

Fig. 2 is a schematic representation of the acetabular cup assembly with the position of the femoral head shown for illustrative purposes.

Fig. 3 is a cross-sectional view of the acetabular cup assembly of the invention.

Fig. 4 is a cross-sectional view of a second embodiment of the acetabular cup assembly of the invention.

Fig. 5 is a cross-sectional view of a third embodiment of the acetabular cup assembly of the invention.

### Description of the Preferred Embodiments

The preferred embodiments herein described are not intended to be exhaustive or to limit the invention to the precise forms disclosed. Rather, they are chosen and described to best explain the invention so that others skilled in the art might utilize their teachings.

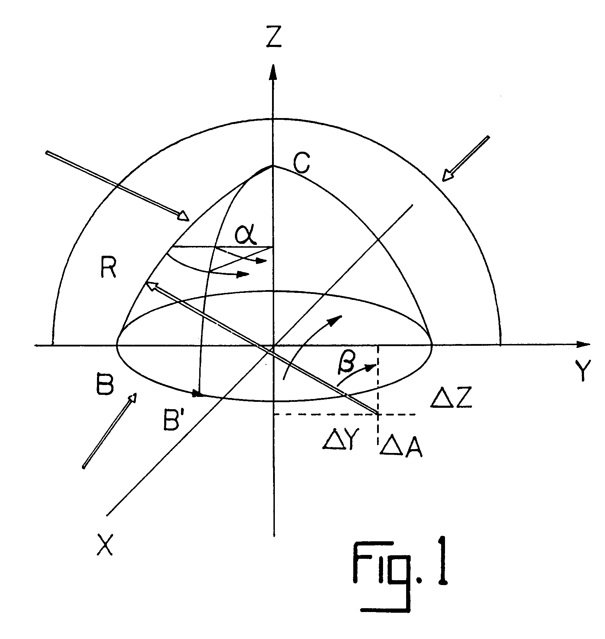

Referring to Figure 1, the coordinate axes **xyz** intersect at the spherical center of the acetabular cup 10. Cup 10 is shown schematically and not to be construed as a limiting configuration of the invention. A point "**A"** is translated in the fourth quadrant of the **yz** plane by some values **Δy** and **Δz**. An arc 12 is struck in the **yz** plane, intersecting the **y** axis at one terminus **"B"** and the **z** axis at its other terminus **"C"**. When arc 12 is rotated about axis **z** through an angle **α** as shown in Figure 1, it becomes the generatrix of the invention's articulating surface. The length of the generatrix is determined by the displacements **Δy** and **Δz** and the length of radius **R**. Figure 1 shows the generatrix having moved through **α** degrees to point **B'** in the **xy** plane. When the generatrix has been rotated through **α** = 2**π** radians, it will have generated the contiguous aspheric articulating surface of the invention.

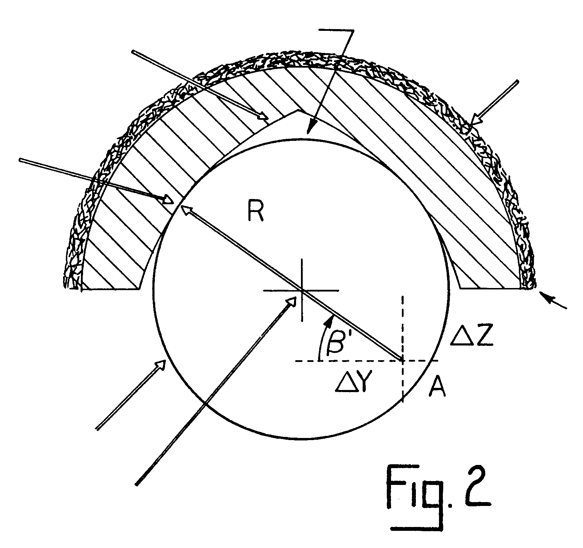

Figure 2 illustrates in diagram form a prosthetic femoral head 14 and the acetabular cup 10 of the invention. The radius of the generated surface **R** is shown at the angle **β'**. At this angle, dictated by the displacements **Δy** and **Δz**, the radius passes through the spherical center 16 of the cup and femoral head, intersecting at the cup-head tangency point 18. This tangency point 18, in the solid embodiment of the invention, is a line that defines and statically isolates a superior space 20 which acts as a lubricant reservoir. The gaps 22 between the cup 10 and head 14 leading toward and away from the line of tangency are equiangled. This is an aid in the establishment of a more or less stable lubricating film between the two surfaces.
Both the radius of the cup generating arc 12 and the displacement of its center are functions of the selected prosthetic head diameter and the selected gap angles leading toward and away from the tangency line contact 18 between the head 14 and cup interior 11. A limiting special case of the present invention is the embodiment in which the displacements **Δy** and **Δz** = 0 creating a cup with the same diameter as the articulated head. In this case, a superior space would have to be created to act as a lubricant reservoir. The opposite limiting special case is the embodiment in which the displacement **Δz** is equal to 0 and **Δy** is infinity. In this case the cup is in the form of an infinite cone equivalent to a cylinder with the same diameter as the articulating head.
The cup and head geometries described above fabricated with combination of various Co-Cr-Mo alloys (ASTM F-75, ASTM F-90, ASTM F-799, Haynes Stellite 6B, Ultimet Alloy, etc.), Titanium Alloys (ASTM F-136, Beta titanium alloys, etc.), Stainless Steels (ASTM F-138, ASTM F1314, etc.), Ceramics (Alumina, Zirconia, Silicon Carbide, Silicon Nitride, etc.), Zirconium Base Alloys, Tungsten Base Alloys, Tantalum and Tantalum Base Alloys, Niobium and Niobium Base Alloys, metal-matrix composites, ceramic matrix composites, polymer matrix composites and polymers. The metals used here are preferably ion-implanted, nitrided, or surface hardened by other commercially available surface hardening process such as surfacing, Titanium nitride coating, diamond-like carbon coating, zirconium oxide coating and other ceramic coatings to improve wear properties of articulating surfaces of the components. The exterior of this cup preferably includes a porous coating which may be fiber metal, beads or plasma spray coated for bony ingrowth application or for mechanical fixation. The porous coating 24 is illustrated in diagrammatical form only.

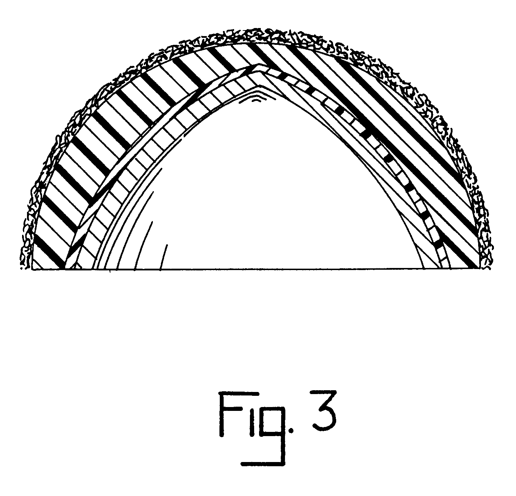

Figure 3 depicts another embodiment of the acetabular cup 10 described in Figure 2. A thin shell 26 is attached to shell 28. The shell 26 is attached to shell 28 by metallurgical bonding or polymer bonding. Fig. 3 illustrates a thin layer of polymer 30 between shells 26 and 28 adhesively bonding the shells together. The materials used for shell 26 and 28 are any one or combination of materials used in the preceding paragraph of this text. The exterior of the cup may also include a porous surface layer 24' for bony ingrowth application as described above.

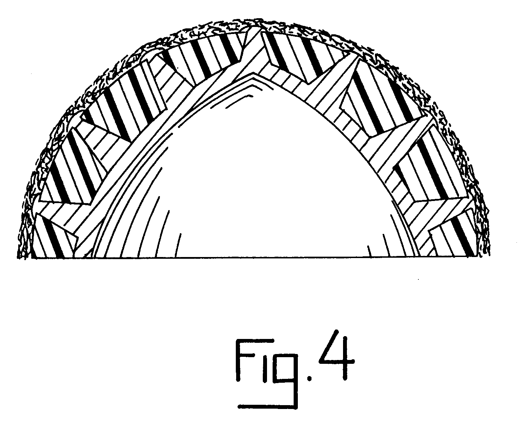

Another embodiment of the acetabular cup is described in Figure 4. Here cup 10'' includes a shell 26' which is mechanically joined to shell 28' by rivets 34 or rivets 34 and metallurgical bonding. In this case also the materials used for shell 26' and 28' can be any one or a combination of materials revealed in the preceding section of this description. A porous coating 24'' as in other designs can also be applied to this design.

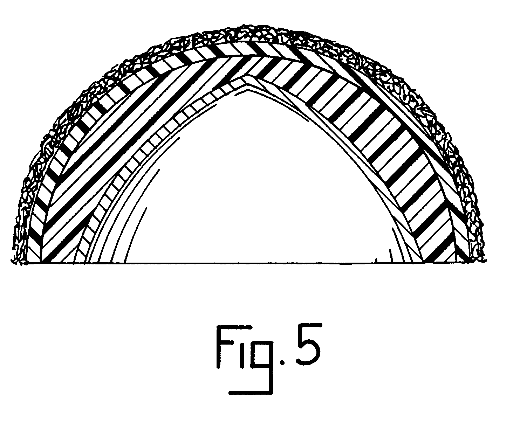

Yet another embodiment of the acetabular cup is depicted in Figure 5. As illustrated, cup 10''' includes metal shells 26'' and 28'' which are attached to a polymer or composite shell 36. The resulting composite structure will provide lower modulus and adequate flexibility to cup 10'''. This design will help in maintaining an adequate lubrication between articulating surfaces of the cup and a femoral head (not shown). The metals used in the fabrication of this cup can be any one or combinations of materials described in the previous section. The materials used for the polymer or composite shell can be Ultra High Molecular Weight Polyethylene (UHMWPE), Ultrapek, PEEK, Bone Cement (Polymethyl methacrylate), or any other polymers or composites used for orthopedic application.

The general geometry described above in the various alloys, non-metallic materials, and composites, are designed to have a specific cup flexibility to enhance the flow of lubricating fluid over the articulating surface. Flexural modulus of the cup plays a role in the establishment and maintenance of a lubricating film. Under the influence of the bimodal force curve of a normal walking step, the femoral head is forced upward into the cup. The resultant compressive force of the head against the reservoir of lubricant in the superior space above the head forces the lubricant out between the head and cup wall. The pressure of the relatively incompressible fluid forces the cup wall away from the head and allows for the establishment of the lubricating film between them. The lower the flexural modulus of the cup material, the thicker the lubricating film.

The general geometry, fabrication materials, design flexibility, surface flexibility, and modification processes are imposed on the articulating surfaces, and geometrically altered to provide discontinuities and channels and other means for the establishment of a stable or semi-stable lubricating film on the surface of the cup and head. These surface features are used in the general cup geometry described in this patent application. It can also be used in a limiting special case of the present invention in which the displacements **Δy** and **Δz** = 0 to create a cup with the same diameter as the articulating head.

## Claims

1. A prosthetic cup prosthesis including a non-spherical surface adapted to accommodate a spherical ball therein for articulating movement against the surface, the surface of the cup being defined by revolution of on arc intersecting an equatorial plane and a polar axis of the cup, the arc having a center of curvature translated from a spherical center of the cup, the arc being rotated about the polar axis for the cup.

2. A prosthetic cup prosthesis configured for accommodating a spherical ball therein for articulating movement against a non-spherical articulating surface of cup, the cup being defined by coordinate x, y, and z, axes which intersect to define a spherical center of the cup, the articulating surface being defined by an arc struck in an yz plane and intersecting the y axis and the z axis, to define the articulating surface the arc is rotated about the z axis 360 degrees, the arc having a center of curvature translated from the spherical center of the cup.

3. The prosthetic cup of Claim 2 wherein a space is formed between the non-spherical articulating surface of the cup and the spherical ball, said space accommodating a quantity of a lubricous substance therein.

4. A prosthetic cup prosthesis having a substantially spherical outer shell and a substantially non-spherical inner shell, wherein the inner shell is configured to accommodate a substantially spherical head for articulating movement therein.

5. The prosthetic cup of Claim 4 wherein the outer shell includes a spherical center defined by intersecting x, y, and z axes, the inner shell includes a non-spherical articulating surface which is defined by an arc intersecting the y and z axes and rotated about the z axis.

6. The prosthetic cup of Claim 4 wherein the inner and outer shells of the cup are formed from metal and are metallurgically bonded together.

7. The prosthetic cup of Claim 4 wherein the inner and outer shells of the cup are formed from metal and adhesively bonded together by an adhesive polymer.

8. The prosthetic cup of Claim 4 wherein the inner and outer shell of the cup are formed from metal and are riveted together.

9. The prosthetic cup of Claim 4 wherein the inner and outer shells of the cup are formed from metal and are spaced apart by a polymer shell.

10. The prosthetic cup of Claim 4 wherein a quantity of lubricous material is carried within a space defined between the non-spherical inner shell and the substantially spherical head.
